(19) **Europäisches Patentamt / European Patent Office / Office européen des brevets**

(11) **EP 4 412 517 B1**

(12) **FASCICULE DE BREVET EUROPEEN**

(45) Date de publication et mention
de la délivrance du brevet:
**03.12.2025 Bulletin 2025/49**

(21) Numéro de dépôt: **21814827.8**

(22) Date de dépôt: **08.10.2021**

(51) Classification Internationale des Brevets (IPC):
**A61B 5/0205** (2006.01)   **A61B 5/369** (2021.01)
**A61B 5/00** (2006.01)

(52) Classification Coopérative des Brevets (CPC):
**A61B 5/0205; A61B 5/369; A61B 5/7264;**
**A61B 5/7275;** A61B 5/4821; A61B 5/4848

(86) Numéro de dépôt international:
**PCT/FR2021/051756**

(87) Numéro de publication internationale:
**WO 2023/057691 (13.04.2023 Gazette 2023/15)**

(54) **PROCEDE MULTIMODAL DE DETECTION D'UN CHANGEMENT D'ETAT PHYSIOLOGIQUE D'UN PATIENT ET DISPOSITIF DE SURVEILLANCE D'UN PATIENT POUR LA MISE EN OEUVRE D'UN TEL PROCEDE**

MULTIMODALES VERFAHREN ZUR ERKENNUNG EINER VERÄNDERUNG DES PHYSIOLOGISCHEN ZUSTANDES EINES PATIENTEN UND VORRICHTUNG ZUR ÜBERWACHUNG EINES PATIENTEN ZUR IMPLEMENTIERUNG SOLCH EINES VERFAHRENS

MULTIMODAL METHOD FOR DETECTING A CHANGE IN A PATIENT'S PHYSIOLOGICAL CONDITION AND DEVICE FOR MONITORING A PATIENT SO AS TO IMPLEMENT SUCH A METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(43) Date de publication de la demande:
**14.08.2024 Bulletin 2024/33**

(73) Titulaires:
- **INSTITUT NATIONAL DE LA SANTE ET DE LA RECHERCHE MEDICALE (INSERM)**
  **75013 Paris (FR)**
- **Institut du Cerveau et de la Moëlle Épinière**
  **75013 Paris (FR)**
- **Centre National de la Recherche Scientifique**
  **75016 Paris (FR)**
- **Assistance Publique - Hôpitaux de Paris**
  **75012 Paris (FR)**
- **Sorbonne Université**
  **75006 Paris (FR)**

(72) Inventeurs:
- **SIMILOWSKI, Thomas**
  **75651 Paris CEDEX 13 (FR)**

- **NAVARRO-SUNE, Xavier**
  **75651 Paris CEDEX 13 (FR)**
- **CHAVEZ, Mario**
  **75651 Paris CEDEX 13 (FR)**
- **RAUX, Mathieu**
  **75651 Paris CEDEX 13 (FR)**

(74) Mandataire: **Gevers & Orès**
**Immeuble le Palatin 2**
**3 Cours du Triangle**
**CS 80165**
**92939 Paris La Défense Cedex (FR)**

(56) Documents cités:
**WO-A1-2013/164462    WO-A1-2016/075324**
**US-A1- 2011 004 110**

• MANOLOVA AGATA ET AL: "Combined EEG and EMG fatigue measurement framework with application to hybrid brain-computer interface", 2016 IEEE INTERNATIONAL BLACK SEA CONFERENCE ON COMMUNICATIONS AND NETWORKING (BLACKSEACOM), IEEE, 6 June 2016 (2016-06-06), pages 1 - 5, XP033085273, DOI: 10.1109/BLACKSEACOM.2016.7901569

• NAVARRO-SUNE X ET AL: "Riemannian Geometry Applied to Detection of Respiratory States From EEG Signals: The Basis for a Brain-Ventilator Interface", IEEE TRANSACTIONS ON BIOMEDICAL ENGINEERING, IEEE, USA, vol. 64, no. 5, 1 May 2017 (2017-05-01), pages 1138 - 1148, XP011646213, ISSN: 0018-9294, [retrieved on 20170418], DOI: 10.1109/TBME.2016.2592820

## Description

### Domaine technique de l'invention

L'invention est relative aux procédés de détection d'un changement d'état physiologique d'un patient par rapport à un état physiologique de référence. L'invention concerne en outre un dispositif de surveillance d'un patient pour la mise en œuvre d'un tel procédé.

La mise au point de l'invention s'inscrit dans une démarche d'amélioration de la caractérisation de l'inconfort respiratoire. L'invention propose ainsi une solution pour améliorer la caractérisation des changements d'état physiologique, notamment des changements de l'état respiratoire d'un patient en anesthésie-réanimation.

### Arrière-plan technique

L'évaluation de l'état respiratoire des patients est menée de manière routinière dans une démarche générale de diagnostic de l'état du patient. Cette évaluation est dite « préopératoire » lorsqu'elle est menée avant l'intervention, « peropératoire » lorsqu'elle est menée pendant l'intervention et « post-opératoire » lorsqu'elle est menée après l'intervention sur le patient. Elle est d'une importance critique pour détecter les éventuelles dyspnées survenant chez le patient au cours de ces différentes phases cliniques. Bien entendu, il existe de nombreuses autres applications qui requièrent une surveillance de l'état respiratoire d'un patient, par exemple la détection de troubles de la respiration pendant le sommeil.

La fonction respiratoire humaine est régulée de façon autonome au niveau du tronc cérébral. Toutefois, en cas de perturbation du débit ventilatoire normal du patient, traduisant souvent une gêne respiratoire pour le patient, certaines régions corticales peuvent être sollicitées. Ces activations corticales, responsables d'un changement d'état respiratoire chez le patient, ont pour but de recruter les muscles respiratoires auxiliaires afin de compenser tout débit ventilatoire inadéquat. On parle de « recrutement musculaire » car l'action musculaire est déclenchée par les signaux nerveux associés aux activations corticales. Une perturbation dans le débit ventilatoire normal du patient entraîne donc une série de modifications visant à réguler différentes constantes physiologiques, appelées modifications homéostatiques, qui sont susceptibles d'être mesurées par des signaux biomédicaux.

Il s'agit par exemple de la présence d'une signature spécifique dans l'électroencéphalogramme (EEG) du patient, d'une intensification de l'activité électromyographique (EMG) des muscles ventilatoires du patient et/ou d'un changement de dynamique dans les tracés respiratoires. Il peut également s'agir d'un changement dans l'activité cardiaque - mesurée par électrocardiogramme (ECG) - du patient puisqu'un changement dans l'état respiratoire de ce dernier peut également déclencher une réponse de stress. De même, un changement peut être observé dans la réponse électrodermale du patient.

L'art antérieur se borne, au choix, à ne s'intéresser qu'à l'analyse et au traitement d'un seul type de données physiologiques (monomodal) pour caractériser les changements d'état physiologique du patient, ou, lorsque plusieurs types de données physiologiques (multimodal) sont analysées pour caractériser les changements d'état physiologique du patient, à les exploiter séparément.

On connait de l'état de la technique de nombreux procédés de détection d'un(de) changement(s) d'état physiologique du patient qui ne sont basés que sur l'analyse et l'exploitation d'un seul type de signal physiologique.

De tels procédés sont par exemple connus des documents US5820560 A, US2010252038 A1 ou encore WO2013164462 A1. Les procédés décrits se basent, selon le cas, uniquement sur les données liées à l'activité électromyographique des muscles respiratoires du patient ou sur les données électro-encéphalographiques de celui-ci. Les procédés monomodaux (basés sur l'information d'un seul type de signaux) décrits dans ces documents requièrent une étape pour confirmer qu'un changement dans le signal observé correspond bien à un changement réel d'état physiologique du patient, selon le signal mesuré. Ceci nécessite de pouvoir préalablement associer le moment de survenu d'un changement physiologique dans le(s) signal(aux) mesuré(s) avec l'évènement physiologique lui-même, sachant que le(s) signal(aux) mesuré(s) en question peut(vent) être très bruité(s). L'étape de confirmation précitée peut donc requérir d'avoir à utiliser plusieurs capteurs différents ou tout autre moyen de mesure pour l'analyse d'un même type de modification homéostatique afin d'identifier plus précisément le moment de l'évènement physiologique.

En outre, toujours dans le but de confirmer un changement réel dans l'état physiologique du patient, il peut être nécessaire avec les procédés de l'art antérieur d'appliquer des traitements complexes aux signaux enregistrés.

C'est par exemple le cas dans le document US5820560 A, dans lequel une série de traitements est nécessaire afin d'en extraire l'information pertinente dans des électromyogrammes. Ce document signale lui-même (Col. 5, l. 51 et suivantes) la difficulté à maintenir le niveau de bruit dans les signaux aussi bas et constant que possible. Des amplificateurs de signaux et des convertisseurs de signaux sont donc utilisés pour compenser les effets du mouvement du diaphragme. De très nombreux capteurs sont également requis.

Les solutions proposées dans ces procédés monomodaux ne limitent pas suffisamment la survenue des fausses alarmes du dispositif d'assistance respiratoire et/ou sont trop complexes à mettre en œuvre. En outre, un autre inconvénient des procédés monomodaux réside dans le fait que, de manière intrinsèque, ils ne permettent d'observer qu'un paramètre physiologique du patient, ce qui ne permet pas de caractériser l'état général du patient.

Il a donc été également proposé des procédés multimodaux combinant plusieurs signaux physiologiques pour la détection d'un changement d'état physiologique du patient.

Un exemple d'un tel procédé est décrit dans le document WO 2013/140229 A1. Ce document divulgue un procédé de régulation d'un dispositif d'assistance respiratoire mécanique. Selon l'approche utilisée dans ce document, l'assistance respiratoire du patient est régulée en fonction de l'évolution du rapport Paw/Eadi, où le paramètre Paw est la valeur du signal due à la pression ventilatoire exercée, tandis que le paramètre Eadi est la valeur du signal électrique causée par l'activité du diaphragme du patient. Le paramètre Paw représente l'activité respiratoire spontanée du patient et est ainsi associé à la pression musculaire Pmusc. Le paramètre Eadi représente quant à lui l'activité respiratoire neuronale qui est à l'origine des mouvements du diaphragme. C'est à partir du rapport Paw/Eadi qu'est régulée l'assistance ventilatoire fournie au patient. Ce rapport permet de déduire la proportion dans laquelle le patient contribue à la respiration par rapport à la respiration totale générée par lui-même et par le dispositif d'assistance ventilatoire. Il est donc question de fournir une estimation de la mesure dans laquelle le patient intervient dans la respiration pour ajuster l'assistance respiratoire. La mesure dans laquelle le patient contribue à la respiration ne permet pas de caractériser un changement de son état physiologique. En effet, un certain nombre de mouvements respiratoires du patient peuvent venir du patient lui-même sans que cela ne soit lié à une gêne respiratoire. En outre, ce procédé multimodal n'utilise pas les corrélations entre les deux paramètres physiologiques précités mais extrait une seule information à partir des mesures des signaux physiologiques associés à ces deux paramètres physiologiques.

Le document US 2004/0254493 décrit une méthode de détection des changements d'état physiologique du patient basée sur l'analyse d'une pluralité de signaux physiologiques. Ce document s'intéresse plus spécifiquement à la détection des troubles de la respiration survenant pendant le sommeil d'un patient. La méthode de détection est basée sur l'analyse des changements se produisant dans les signaux électro-encéphalographiques durant le cycle respiratoire de l'individu. Cette méthode de détection fait également intervenir, en plus de l'analyse d'électro-encéphalogrammes, l'analyse d'autres signaux caractérisant l'état physiologique du patient tels que des électro-oculogrammes, des électromyogrammes, des mesures d'écoulement d'air oral et nasal, etc. Toutefois, la méthode se borne à prévoir l'enregistrement simultané des différents signaux sans opérer de traitement permettant de tirer avantage des éventuelles complémentarités liées à l'acquisition conjointe de ces différents signaux. En effet, seules les données issues des signaux électro-encéphalographiques font l'objet d'un traitement particulier. Le praticien reçoit donc des données brutes qu'il doit lui-même interpréter. La méthode n'est donc pas adaptée pour permettre une modification en temps-réel de l'assistance respiratoire du patient. Ceci découle vraisemblablement du fait que la surveillance de l'état respiratoire des patients atteints de troubles se manifestant durant la phase de sommeil vise généralement à poser un diagnostic, le traitement intervenant après.

## Résumé de l'invention

L'invention permet de surmonter les inconvénients des procédés connus de l'art antérieur en exploitant et corrélant les données issues de différents signaux physiologiques caractérisant l'état du patient afin de mieux détecter et caractériser un changement dans l'état du patient. L'invention est définie par les présentes revendications 1 à 12.

À cet égard, l'invention concerne un procédé de détection d'un changement d'état physiologique d'un patient par rapport à un état physiologique de référence associé à une matrice de référence $X_{ref}$ de signaux $X_{ref,i}$ (i = [1...N+1], N entier) et à une période de référence $W_0$, le procédé mettant en œuvre en boucle les étapes suivantes :

A) effectuer dans M segments temporels m d'une fenêtre d'observation des mesures de signaux $S_1$ électro-encéphalographiques du patient selon *n* voies et à *p* instants pour générer M matrices de mesures $X_{1,m}$ (m $\varepsilon$ [1...M], M entier) comportant n*p échantillons et simultanément, effectuer des mesures d'au moins N autres signaux physiologiques $S_{N+1}$ (N $\geq$ 1) du patient, différent des signaux $S_1$ électro-encéphalographiques, pour générer N autres matrices de mesures $X_{i}$=2 ... $N_{+}$1,m,

C) pour chaque segment temporel m de la période de référence $W_0$, déterminer des distances $d_i(m)$ entre chaque signal $X_{i,m}$ mesuré et la composante $X_{ref,i}$ de la période de référence,

D) transformer les distances $d_i(m)$ calculées à l'étape C) via une fonction log d(m),

E) choisir la précision des valeurs obtenues à l'issu de l'étape D en sélectionnant un quantile de référence $u_0$ sur l'ensemble des valeurs de la période de référence $W_0$ et associer à chaque distance $d_i(m)$ une variable scalaire $p_{i=1,...,N+1}$ ($0<p_{i=1,...,N+1}<1$) dépendant du quantile de référence *u*,

F) fusionner les données obtenues à l'issu de l'étape E) en effectuant une somme pondérée des variables $p_{i=1,...,N+1}$ pour obtenir des distances résultantes $d_{fusion}(m)$,

G) déterminer un écart e(m) à l'état physiologique de référence en fonction des distances $d_{fusion}(m)$.

L'invention propose ainsi un procédé multimodal de caractérisation de l'état physiologique d'un patient dans lequel les données issues des différents types de signaux physiologiques mesurés sont traitées et fusionnées de manière à en déduire un changement d'état physiologique du patient. L'invention va ainsi au-delà des procédés de l'art antérieur et permet d'obtenir des variables réduites résultant de la combinaison des données acquises par les différents signaux. L'information obtenue est donc plus qualitative, plus fiable et plus précise en comparaison de ce que permet l'art antérieur. L'analyse de différents signaux physiologiques $S_1,..., S_{N+1}$ permet de mieux caractériser l'état du patient. La fusion des données issues de ces différents signaux permet de réduire, voire de supprimer, les segments de données non-qualitatifs, de minimiser le poids des artéfacts dans les données finales ce qui permet d'avoir une information de meilleure qualité et permet donc de réduire les fausses alarmes du dispositif d'assistance médicale. L'invention propose ainsi une solution de surveillance en temps réel qui agit automatiquement sur le dispositif d'assistance respiratoire et permet de réduire les interventions inutiles du praticien sur le patient.

Selon différentes caractéristiques de l'invention qui pourront être prises ensemble ou séparément :

- lors d'une étape B) on centre et on filtre les signaux $S_1$ électro-encéphalographiques dans Q bandes de fréquence prédéterminées pour obtenir MxQ matrices de mesures filtrées $X_{1,m,q}$ (q ε [1...Q]) et on détermine MxQ matrices normées de covariance spatiale par la formule :

$$C_{m,q} = \frac{X_{1,m,q}X_{1,m,q}^T}{trace\,(X_{1,m,q}X_{1,m,q}^T)}\ ;$$

- lors de l'étape C), on détermine des distances riemanniennes $d_{i,r}(m)$ entre chaque matrice normée de covariance spatiale $C_{m,q}$ et les composantes $X_{ref,i}$ de la matrice de référence associées aux mesures électro-encéphalographiques ;
- lors de l'étape C), on détermine des distances statistiques $d_{i,s}(m)$ entre un(des) signal(aux) $X_{i,m}$ mesuré(s), autres que les signaux électro-encéphalographiques, et la(les) composante(s) $X_{ref,i}$ de la période de référence qui lui est(sont) associée(s) ;
- $d_{fusion}(m)$ est calculée selon la relation suivante :

$$d_{fusion}(m) = \sum_i \lambda_i p_{i=1,...,N+1}(m),$$

avec

$$p_{i=1,...,N+1}(m) = \frac{1}{1 + e^{u_0} - \log(d_{i=1,...,N+1}(m))}$$

où $\lambda_i$ est le poids affecté à chaque variable $p_{i=1,...N+1}$, les poids $\lambda_i$ vérifiant $\sum_i \lambda_i = 1$, $u_0$ est le quantile de référence, $d_{1,q}(m)$ sont les distances riemanniennes et $d_{i>1}(m)$ sont les distances statistiques ;
- les poids $\lambda_i$ sont sélectionnés selon la qualité du signal $S_i$ auquel ils sont associés ou selon l'importance du signal $S_i$ auquel ils sont associés pour déterminer un changement d'état physiologique chez le patient ;
- une étape de filtrage des distances résultantes $d_{fusion}(m)$ est mise en œuvre préalablement à l'étape F) ;
- le filtrage consiste en un lissage obtenu par une moyenne mobile sur les L derniers segments de la fenêtre d'observation (1<L<M) appliquée aux distances résultantes $d_{fusion}(m)$, ladite distance $d_{fusion}(m)$ étant alors calculée selon la relation suivante :

$$d_{fusion}^L(m) = \frac{1}{L}\sum_{i=0}^{L-1} d_{fusion}(m - i)$$

- les L derniers segments sont choisis de telle sorte que la moyenne mobile soit calculée en ne tenant compte des signaux mesurés qu'avec un retard compris entre 10 secondes et 1 minute, de préférence entre 30 secondes et 1 minute par rapport au début de la fenêtre d'observation ;
- lesdits autres signaux physiologiques $S_i$ sont l'activité cardiaque et/ou l'activité respiratoire et/ou l'activité musculaire et/ou un signal lié au mouvement ;
- le procédé comprenant en outre une étape au cours de laquelle on compare chacun des écarts e(m) à un seuil $\Theta$ déterminé.

L'invention concerne en outre un dispositif de surveillance d'un patient pour la mise en œuvre d'un procédé tel que précédemment décrit, le dispositif comportant :

- des moyens de mesure de signaux électro-encéphalographiques pour mesurer des signaux électro-encéphalographiques du patient,
- des moyens de mesure de l'activité cardiaque et/ou de l'activité respiratoire et/ou de l'activité musculaire et/ou d'un mouvement,
- des moyens de traitement de signal en temps réel, lesdits moyens comprenant au moins des moyens de détermination des composantes $X_{i,ref}$ de la matrice de référence, des moyens de calcul des écarts e(m) à la situation de référence et des moyens de fusion des données.

L'invention concerne enfin un dispositif d'assistance médicale comportant un dispositif de surveillance tel que précédemment décrit.

## Brève description des figures

D'autres objets et caractéristiques de l'invention apparaîtront plus clairement dans la description qui suit, faite en référence aux figures annexées, dans lesquelles :

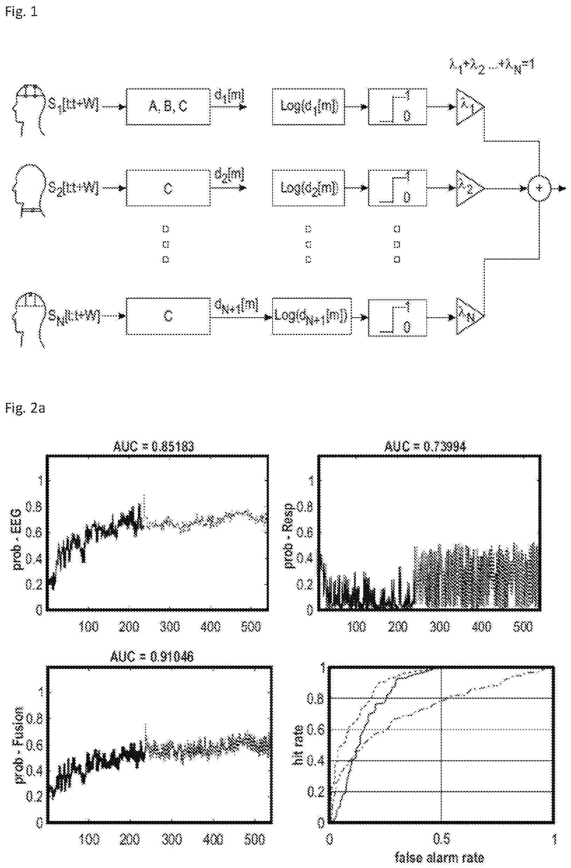

- La figure 1 est un schéma de principe illustrant certaines étapes du procédé de détection selon l'invention depuis l'extraction des données de différents signaux physiologiques jusqu'à la fusion desdites données ;
- La figure 2a illustre les performances de trois détecteurs pour un sujet SU1 : la première figure en partant de la gauche illustre les performances (AUC) d'un détecteur indépendant exploitant les signaux $S_1$ électro-encéphalographiques seuls, la deuxième figure en partant de la gauche illustre les performances (AUC) d'un détecteur indépendant exploitant les signaux $S_2$ respiratoires seuls et la troisième figure en partant de la gauche illustre les performances (AUC) d'un détecteur exploitant les distances fusionnées $d_{fusion}$, la quatrième figure en partant de la gauche (et donc la figure la plus à droite) illustre les courbes ROC de ces trois détecteurs ;

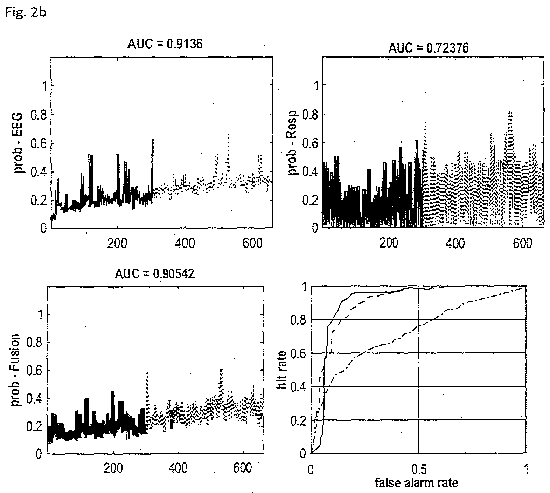

- La figure 2b diffère de la figure 2a en ce que les performances sont illustrées pour un sujet SU2 ;

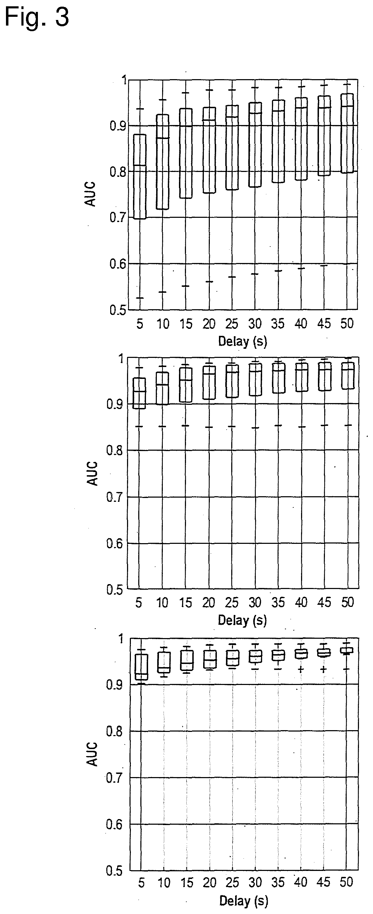

- La figure 3 illustre les effets d'une étape de filtrage (lissage) sur les performances de différents détecteurs, notamment un détecteur indépendant exploitant les signaux $S_2$ respiratoires seuls, un détecteur indépendant exploitant les signaux $S_1$ électro-encéphalographiques seuls et un détecteur exploitant les distances fusionnées $d_{fusion}$ (dans cet ordre en lisant de gauche à droite).

## Description détaillée de l'invention

<u>Description du mode général du procédé de détection selon l'invention</u>

L'invention concerne un procédé de détection d'un changement d'état physiologique d'un patient. L'état physiologique du patient se caractérise notamment par un ensemble de fonctions et de réactions du patient, appelées également paramètres physiologiques. Dans la suite, on distinguera un état physiologique de référence d'un état physiologique modifié, ce dernier état caractérisant une modification de l'état physiologique du patient par rapport à son état physiologique de référence. Une modification de l'état physiologique du patient est considérée comme une modification de son état physiologique par rapport à un état physiologique de référence du patient, lorsque cette modification est due à au moins une modification homéostatique définie quelques paragraphes plus loin.

L'état physiologique de référence n'est pas nécessairement l'état physiologique du patient lorsqu'il est sain, à savoir l'état physiologique dans lequel se trouve le patient lorsque ses fonctions et réactions sont normales et/ou que le patient ne présente aucune pathologie. L'état physiologique de référence est l'état physiologique constaté du patient à partir d'un instant donné, que le patient soit en bonne ou en mauvaise santé, la constatation étant associée à un certain nombre de mesures, elles-mêmes associées à des signaux qui seront décrits plus en détail dans la suite.

L'état physiologique modifié du patient n'est pas nécessairement l'état physiologique du patient lorsqu'il est malade, à savoir l'état physiologique dans lequel se trouve le patient lorsque ses fonctions et réactions sont anormales et/ou que le patient présente une ou plusieurs pathologies. Un état physiologique modifié du patient correspond à un état

physiologique divergent de l'état physiologique de référence. Ceci se produit lorsqu'au moins une ou, en pratique, plusieurs des fonctions ou des réactions du patient dévie(nt) des fonctions et ou réactions que présente le patient à l'état physiologique de référence. Dans ce cas, on parle de modification homéostatique.

Un changement d'état physiologique ne se réside donc pas dans le simple fait que le patient sain devient malade ou inversement, mais réside dans tout changement qui est de nature à le faire passer de l'état physiologique de référence à un autre état suite à une modification homéostatique. Autrement dit, dans le cadre de l'invention, l'état physiologique du patient ne présume pas de la bonne santé ou non du patient.

Dans le cadre de l'invention, l'état physiologique de référence est quant à lui associé à une matrice $X_{ref}$, dite matrice de référence, construite à partir d'une pluralité de composantes $X_{ref,i}$ ($i = [1...N+1]$, N entier). Chaque composante $X_{ref,i}$ de la matrice de référence $X_{ref}$ est associée à une mesure de dimension donnée, dont les éléments sont les différents signaux physiologiques du patient à l'état physiologique de référence. Cet état physiologique de référence est associé à une période de référence $W_0$. Les dimensions de la matrice de référence dépendent donc : 1) du nombre de signaux physiologiques mesurés indépendamment du type de signal physiologique auquel le signal mesuré est effectivement affecté et, 2) du nombre d'instants auxquels les mesures des signaux physiologiques sont subséquemment effectuées, c'est-à-dire les mesures effectuées hors de l'état physiologique de référence qui, lui, n'est associé qu'à la période de référence $W_0$.

En ce qui concerne le point 1) ci-dessus, une composante $X_{ref,i}$ de la matrice de référence peut notamment être associée à des mesures spatio-temporelles, notamment lorsque l'évaluation du paramètre physiologique nécessite que des mesures soient effectuées dans différentes régions du corps ou d'une zone du corps du patient en fonction du temps (par ex. différents signaux électro-encéphalographiques ou différents signaux musculaires enregistrés simultanément). De telles mesures sont fréquemment réalisées lorsqu'il s'agit d'évaluer l'activité corticale ou encore musculaire du patient. Elles font intervenir une série d'électrodes, le cas échéant d'aiguilles, placées dans des zones appropriées du corps du patient afin de cartographier, et donc représenter spatialement l'évolution du paramètre physiologique concerné. Par exemple, des mesures électro-encéphalographiques faisant intervenir 10 électrodes sont donc associées à 10 signaux physiologiques différents, un signal physiologique étant affecté à chaque électrode. Si en outre, ces mesures sont mises en œuvre à 8 instants différents, indépendamment de toute segmentation éventuelle, la composante $X_{ref,i}$ de la matrice de référence associée aux mesures électro-encéphalographiques est une matrice de dimension 10 x 8. En revanche, cela ne présume pas des dimensions pour les autres composantes de la matrice $X_{ref}$ de référence qui comprend par ailleurs d'autres signaux $X_{ref,i}$.

En ce qui concerne le point 2) ci-dessus, il est important de rappeler que si l'invention cherche à établir des corrélations entre les différents types de modifications homéostatiques, et qu'en conséquent il est donc essentiel, comme cela sera vu ci-après, que les mesures des différents types de signaux physiologiques soient effectués parallèlement, i.e. simultanément, tout au moins sur une fenêtre temporelle commune, cela ne signifie pas pour autant que la fenêtre temporelle sur laquelle les mesures sont prises doit être de même longueur (nombre d'échantillons ou segments) pour tous les paramètres physiologiques mesurés. Le praticien peut en effet vouloir observer l'évolution d'un ou plusieurs paramètres physiologiques plus longuement que d'autres. On reste dans le cadre de l'invention dès que le procédé est mis en œuvre avec au moins deux types de signaux physiologiques.

La matrice $X_{ref}$ de référence comprend en tout état de cause autant de composantes $X_{ref,i}$ qu'il y a de types de signaux physiologiques observés. Dans l'invention, il y a i = [1...N+1] types de signaux physiologiques mesurés.

Lors d'une première étape A) du procédé de détection selon l'invention on effectue, dans M segments temporels d'une fenêtre d'observation, des mesures électro-encéphalographiques du patient. Les mesures électro-encéphalographiques (EEG) permettent de mesurer l'activité électrique du cerveau du patient et se présentent sous forme d'enregistrements.

La fenêtre d'observation est une fenêtre d'observation temporelle. Elle définit un intervalle de temps pendant lequel sont effectuées les mesures à des instants dont la fréquence peut être prédéterminée. Les M segments temporels définissent des sous-intervalles de temps de la fenêtre d'observation durant lesquelles on effectue les mesures des signaux $S_1$ électro-encéphalographiques de manière continue ou discrète. On affecte à chacun des M segments temporels, une durée notée W. Si la fenêtre d'observation est la période de référence définie précédemment, on affecte de même à chaque segment de la période de référence une durée notée $W_{0,m}$.

On définit p instants auxquels sont réalisées les mesures électro-encéphalographiques dans un segment temporel m de ladite fenêtre d'observation. On définit en outre n voies par l'intermédiaire desquelles les mesures électro-encéphalographique du patient sont effectuées. En pratique, le patient est muni d'un casque comprenant n électrodes positionnées de manière appropriée au niveau de la tête du patient pour mesurer les signaux $S_1$ électro-encéphalographiques, chaque électrode étant associée à une voie. On génère ainsi pour chaque segment temporel M une matrice de dimension n x p, les éléments de la matrice correspondant à des mesures spatio-temporelles. On notera $X_{1,m}$, m $\varepsilon$ [1...M] et M entier, les M matrices de mesure ainsi générées.

Simultanément, toujours au cours de la première étape A) du procédé selon l'invention, on effectue des mesures d'au moins un autre type de signal physiologique du patient. En effet, comme indiqué précédemment, le procédé selon l'invention est un procédé multimodal de détection d'un changement d'état physiologique. À ce titre, le procédé selon l'invention est basé sur l'analyse simultanée de plusieurs paramètres physiologiques du patient. Il suffit qu'au moins un autre type de paramètre physiologique, différent de l'activité corticale (EEG), soit observé pour que le procédé soit considéré comme étant un procédé multimodal. L'autre (les autres) signal(aux) physiologique(s) mesuré(s) sont des électromyogrammes, le débit ou le flot respiratoire, des distensions thoraciques ou sons respiratoires, des électrocardiogrammes (ECG), des mesures électrodermales ou des mesures de mouvement. Ceci n'est en rien limitatif et d'autres paramètres physiologiques utiles à l'homme de l'art pour caractériser l'état physiologique du patient peuvent être mesurés.

Dans le cadre de l'invention, on définit au nombre de N les autres types de signaux physiologiques $S_{N+1}$, $N \cong 1$, mesurés autres que les signaux $S_1$ électro-encéphalographiques. Il y a donc N+1 types de signaux physiologiques du patient qui sont mesurés simultanément sur au moins une fenêtre d'observation commune. À l'instar des signaux $S_1$ électro-encéphalographiques, on effectue dans les M segments temporels de la fenêtre d'observation des mesures de(es) l'autre(autres) signal(aux ») physiologique(s) du patient. On génère ainsi, pour chaque segment temporel m, N autres matrices de mesure $X_{i = 2...N+1,m}$. Les dimensions de chaque matrice $X_{N,m}$ dépendent du type de paramètre physiologique considéré.

Les sous-étapes B1), B2) et C1) décrites dans la suite concernent des traitements appliqués aux signaux $S_1$ électro-encéphalographiques selon une mise en œuvre particulière, i.e. non limitative, de la présente invention. Cette mise en œuvre concerne le cas où les signaux $S_1$ électro-encéphalographiques sont traités dans la géométrie riemannienne. On pourra notamment se référer au document WO 2013/164462 A1 qui décrit de telles étapes. Il faut noter que tout autre signal mesuré, i.e. autre que les signaux $S_1$ électro-encéphalographiques, dont ce traitement s'avère plus opportun pourra faire l'objet des traitements décrits dans ces étapes.

Selon une mise en œuvre particulière, lors d'une deuxième étape B) optionnelle, et plus précisément une première sous-étape B1), on centre et on filtre chaque signal de la matrice $X_{1,m}$ dans Q bandes de fréquence prédéterminées pour obtenir MxQ matrices de mesures filtrée $X_{1,m,q}$, $q \, \varepsilon \, [1...Q]$. On rappelle que les matrices de mesure $X_{1,m}$ ont été obtenues par des mesures électro-encéphalographiques. Les opérations de filtrage séquentiel et de centrage des matrices de mesure ont le sens usuel mathématique que l'on donne à ce type d'opérations. Avantageusement, on notera que les matrices de mesure $X_{1,m}$ sont centrées et filtrées dans cinq bandes de fréquence correspondant aux fréquences conventionnellement utilisées pour l'électro-encéphalographie, à savoir 1-4 Hz, 4-8 Hz, 8-12 Hz, 12-24 Hz et 24-48 Hz. Cela permet de conserver uniquement l'information provenant des régions d'intérêt du cerveau du patient, chaque gamme de fréquences étant associée à un ou plusieurs type(s) de mouvements du patient. Par exemple, dans le cas d'une pluralité d'électromyogrammes mesurés à différents endroits du diaphragme, il aurait alors été approprié de sélectionner des bandes de fréquence propres à la dynamique musculaire du diaphragme.

Toujours au cours de la deuxième étape B) optionnelle, une deuxième sous-étape B2) consiste à déterminer des matrices normées de covariance spatiale à partir de la formule suivante :

$$C_{m,q} = \frac{X_{1,m,q} X_{1,m,q}^{\,T}}{trace\,(X_{1,m,q} X_{1,m,q}^{\,T})} \qquad (1)$$

où $C_{m,q}$ sont les matrices normées de covariance spatiale, $X_{1,m,q}$ sont les matrices de mesures filtrées et $X_{1,m,q}^{\,T}$ sont les matrices transposées correspondantes. Les matrices $C_{m,q}$ de covariance spatiale caractérisent la synchronisation des activités corticales au cours du temps. Elles comportent, dans les éléments de la diagonale, les synchronisations locales et, hors diagonales, les synchronisations à distance qui sont alors les caractéristiques de la dynamique du réseau neuronal.

Selon une mise en œuvre particulière, lors d'une troisième étape C), plus précisément d'une première sous-étape C1) de la troisième étape, on détermine, pour chaque segment temporel m de la période de référence $W_0$, des distances riemanniennes $d_{1,r}(m)$ entre chaque matrice normée de covariance spatiale $C_{m,q}$ et la composante $X_{ref,1}$ de la matrice de référence associée aux mesures électro-encéphalographiques. Plus précisément, on calcule pour chaque segment temporel m de la période de référence $W_0$, la distance riemannienne $d_{1,r}(m)$ entre chaque matrice normée de covariance spatiale $C_{m,q}$ calculée dans le segment temporel m, de durée W, à un l'instant t : t + W et la composante $X_{ref,1}$ de la matrice de référence calculée dans la période de référence $W_0$. À cet égard, dans chaque bande de fréquence, des matrices prototypes $PR_{q,r}$, où $r \, \varepsilon \, [1... R]$ sont déterminées à partir de la répartition des matrices de covariance spatiale $C_{m,q}$. Chaque prototype est un représentant d'une sous-classe de la synchronisation, et est ici estimé par une moyenne de Karcher des matrices de covariance spatiale de référence $C_{m,q}$ de voisinage. Il faut noter que dans le cas d'un seul prototype, celui-ci correspond à la matrice de covariance moyenne pour toute la période de référence.

Une procédure de calcul des prototypes $PR_{q,r}$ selon une mise en œuvre particulière, et donc non limitative, est décrite ci-dessous et est appliquée pour chaque bande de fréquence. Selon cette mise en œuvre particulière, ce calcul repose sur l'algorithme des nuées dynamiques (E. Diday, Une nouvelle méthode en classification automatique et reconnaissance des formes la méthode des nuées dynamiques. Revue de Statistique Appliquée, Tome 19-(1971) no. 2, pp. 19-33) adapté à la métrique riemannienne. On notera en effet qu'en traitement du signal, on utilise habituellement la norme classique de Frobenius pour définir des distances entre des matrices de covariance (qui sont par définition des matrices Hermitiennes définies positives). Cette approche suppose un espace vectoriel normé de courbure nulle. Cependant, l'espace des matrices Hermitiennes définies positives ressort plutôt des espaces métriques à courbure négative. Cette mise en œuvre particulière utilise, de préférence, les outils de la géométrie Riemannienne pour manipuler les matrices de covariance. Dans ce cadre, la distance entre deux matrices correspond à la géodésique dans l'espace engendré par leur propriété Hermitienne, et la moyenne des matrices de covariance ne correspond plus à une moyenne arithmétique comme classiquement, mais à une moyenne géométrique.

```
1 : procédure
   C est un tableau de M matrices de covariance.
   Q est le nombre de prototypes.
   M_R est la moyenne de Karcher d'un ensemble de matrices
2 : k:=M
3 : eps := 10^-8
4: kk =random (k)
5: PR^j:=C_{kk(j)} ; j:=1:Q ; PR_new^j :=0
6 : tant que abs (PR_new^j - PR^j) > eps
7 : PR_new^j : = PR^j ;
8 :      pour i := 1 à k
9 :      label (i) := arg min _{j=1:Q} [dist (PR^j, C^i)]
10 :     fin pour
11 : calcul de la moyenne de Karcher pour chaque partition PR
12 :     pour j := 1 à Q;
13 :     kj= [label (1:k): = j]; PR^j = M_R (C^{kj})
14 :     fin pour
15 : fin tant que
16 : retourner PR
```

Selon cette mise en œuvre particulière, la distance *dist,* i.e. appelée $d_{i,r}$ entre les matrices de covariance est la distance riemannienne suivante : si $P_1$, $P_2$ sont deux matrices, alors :

$$dist\ (P_1, P_2) = \left\{ \sum_{k=1}^{K} ln^2\ (\lambda_k) \right\}^{1/2} \qquad (2)$$

où *dist* $(P_1, P_2)$ est la distance riemannienne entre les matrices prototypes $P_1$ et $P_2$, les $\lambda_k$ sont les K valeurs propres de la matrice conjointe $P_1^{-1}P_2$ .

La distance riemannienne **dist** vérifie les trois propriétés d'une distance, à savoir la symétrie, la séparation et l'inégalité triangulaire.

Selon une mise en œuvre particulière, la moyenne peut être calculée à l'aide d'une procédure de descente de gradient qui converge rapidement (Pennec, Statistical computing on manifolds for computational anatomy. 2006. Thèse de doctorat. Université Nice Sophia Antipolis 2006) :

```
1 : procédure
   C est un tableau de M matrices de covariance.
2: CM := C(1) ; d=0 ;
3: on initialise la moyenne avec la première valeur du tableau.
4: e := 10^-8 ;
5: tant que d > e
6: W := 0 ;
7: pour i = 1 à M
8:      W := W + log_{CM} (C(i)) ;
```

> 9:      on somme les vecteurs tangents dans l'espace tangent
> 10: fin pour
> 11: W : = W/M;
> 12: on revient sur la variété avec la carte exponentielle et on réitère
> 13: CM_new := **exp$_{CM}$** (W) :      distance entre 2 itérations successives
> 14:      d = **dist** (CM_new, CM)
> 15: CM=CM_new
> 16: fin tant que
> 17: **M$_R$** = CM
> 18: retourner **M$_R$** ;

où les opérateurs vérifient :

$$exp_{CM} (W) = CM^{1/2} \exp(CM^{-\frac{1}{2}} W \ CM^{-\frac{1}{2}}) \ CM^{\frac{1}{2}}$$

$$log_{CM} (W) = CM^{1/2} \log(CM^{-\frac{1}{2}} W \ CM^{-\frac{1}{2}}) \ CM^{\frac{1}{2}}$$

Subséquemment ou simultanément, lors d'une deuxième sous-étape C2) de la troisième étape C, on calcule des distances statistiques $d_{i,s}(M)$ entre un(des) signal(aux) $X_{i,m}$ mesuré(s) et la(les) composante(s) $X_{ref,i}$ de la période de référence $W_0$. Plus précisément, on calcule pour chaque segment temporel m, les distances statistiques $d_{i,s}(m)$ entre un(des) signal(aux) $X_{i,m}$ mesuré(s) dans le segment temporel m, de durée W, à un l'instant t : t + W et la composante $X_{ref,i}$ de la période de référence $W_0$. Le(s) signal(aux) concerné(s) est(sont) celui(ceux) qui n'a(ont) pas fait(s) l'objet d'un traitement dans la géométrie riemannienne pour quelque raison que ce soit, par exemple car la géométrie riemannienne n'est pas adaptée ou encore ne s'avère pas adaptée. Dans le cas de cette mise en œuvre particulières, une distance $d_i$ correspond donc soit à une distance riemannienne $d_{i,r}(m)$ ou à une distance statistique $d_{i,s}(m)$ en tenant compte des signaux $S_1$ électro-encéphalographiques et des autres signaux. Le calcul des distances statistiques s'effectue selon le traitement mathématique classique.

Il convient de préciser que les étapes B) et C) peuvent être mise en œuvre sans exploiter les mesures des signaux $S_1$ électro-encéphalographiques dans la géométrie riemannienne. En effet, l'exploitation des signaux $S_1$ électro-encéphalographiques dans la géométrie riemannienne est plus aisée car elle permet de faciliter les mesures réalisées aux différents points de la tête du patient. Toutefois, ce n'est pas obligatoire même si cette méthode donne de meilleurs résultats. Une alternative à la distance riemannienne entre matrices de covariance peut être la distance euclidienne entre les matrices (la norme de Frobenius de la différence algébrique de deux matrices). Une autre alternative consisterait à directement mesurer des distances entre les signaux EEG, sans nécessairement avoir recours aux matrices de covariance. Par exemple, on pourrait avoir recours aux distances statistiques de Hellinger, ou la distance de Bhattacharyya (Basseville, M. (1989). Distance measures for signal processing and pattern recognition. Signal processing, 18(4), 349-369.).

D'autres méthodes décrites dans les documents FR 2 903 314 A ou encore US 2004/0254493 A1, déjà cité dans le préambule de la description, permettent également d'exploiter de tels signaux. Le document FR 2 903 314 A décrit une autre méthode d'exploitation des signaux électro-encéphalographiques dans laquelle les distances correspondent à un potentiel électro-encéphalographique. Dans le document US 2004/0254493 A1, les distances correspondent aux différences entre une puissance de segment EEG maximale et une puissance de segment EEG minimale, tout au long du cycle respiratoire. Ceci est d'importance dans la mesure où l'acquisition des signaux $S_1$ électro-encéphalographiques peut ne pas être disponible, ou s'avérer présenter de nombreux artéfacts.

Plus généralement, les distances peuvent être calculées par toute autre méthode connue de l'art antérieur. En tout état de cause, une « distance » quantifie la différence statistique entre deux valeurs d'un paramètre physiologique. Autrement dit, la distance est donc l'expression mathématique de la modification d'une constant homéostatique à un instant donné et sa valeur dans la période de référence, et qui peut être associée à la survenue d'un changement physiologique chez le patient. Pour déterminer les distances fusionnées $d_{fusion}(m)$, tel que décrit dans la suite de la présente description, il suffit de déterminer des distances entre chaque matrice des mesures effectuées dans les segments temporels m et la composante $X_{ref,i}$ (i = [1 ... N+1]) de la matrice de référence associée. Considérons uniquement le signal $S_2$ de la figure 1 de la présente demande. Un classifieur considéré calcule la distance d'une variable/valeur mesuré(e) à un instant donné, et la distribution de la même variable/valeur calculé(e) dans la période de référence. Plus spécifiquement, il pourrait s'agir d'un classifieur SVM (Machine à Vecteur Support) à une classe ou d'un classifieur basé sur la distance de Mahalanobis. Les classifieurs utilisés pour calculer les distances ne sont donc pas limitatifs.

L'invention ne réside donc pas dans les méthodes employées pour calculer les distances, ni même dans le fait d'utiliser des distances pour fusionner les données issues des différentes mesures mais dans le fait même de fusionner les données, comme cela sera vu plus en détail dans la suite. Ainsi, l'étape C) réside dans le fait de déterminer des distances $d_i$(m) entre chaque signal $X_{i,m}$ mesuré et la composante $X_{ref,i}$ de la période de référence, pour chaque segment temporel m de la période de référence $W_0$, les distances $d_i$(m) pouvant être des distances riemanniennes $d_{i,r}$(m) et/ou des distances statistiques $d_{i,s}$(m) et/ou tout autre type de distances, par exemple du type de celles vues précédemment.

On décrit maintenant, en référence à la figure 1, une mise en œuvre particulière par laquelle s'effectue la fusion des données obtenues à partir des distances riemanniennes et statistiques. La fusion des données qui y est réalisée rend la détection des changements d'état physiologiques plus qualitative, plus fiable et plus précise. En outre, cette fusion permet de mieux caractériser l'état physiologique du patient.

Selon une mise en œuvre particulière, lors d'une quatrième étape D) du procédé, on transforme les distances $d_i$(m) calculées à l'étape C) via une fonction log d(m). Le but de cette étape est de transformer les distances riemanniennes et statistiques de sorte à stabiliser leur variance, et à avoir leurs valeurs comprises entre 0 et 1. En effet, des écarts significatifs peuvent être observés entre les distances calculées au sein des différents segments temporels m, indépendamment du type de distance visé, i.e. distance statistique ou distance riemannienne. Il convient donc de mieux imager mathématiquement les variations relatives entre les différentes distances calculées dans les M segments temporels m, et ce, pour chacune des distances riemanniennes et statistiques. Selon un mode de réalisation préféré, on cherche à rendre les distributions plus proches de la distribution Gaussienne. Toutefois, l'utilisation d'une distribution Gaussienne n'est pas obligatoire et d'autres types de distribution à la portée de l'homme de l'art peuvent être envisagés dans la mesure où ils permettent de stabiliser les variances des données mesurées. À l'issu de l'étape D), on obtient donc les logarithmes log(di(m)) des distances précitées.

Selon cette mise en œuvre particulière, lors d'une cinquième étape E), notamment une première sous-étape E1) de la cinquième étape E), on choisit la précision des valeurs obtenues à l'issu de l'étape D) en sélectionnant un quantile de référence $u_0$ sur l'ensemble des valeurs de la période de référence $W_0$. Autrement dit, lors de cette première sous-étape E1), on choisit le quantile de référence $u_0$ qui sera appliqué à l'ensemble des valeurs mesurées lors de l'étape D) en le sélectionnant sur l'ensemble des valeurs de la période de référence $W_0$.

En pratique, le quantile approprié est choisi d'une part en fonction du nombre de points dans la distribution et d'autre part en fonction de la précision souhaitée pour décrire la distribution, c'est-à-dire pour décrire l'ensemble des valeurs mesurées dans la période de référence $W_0$. D'autres critères tels que le nombre de données aberrantes ou encore le nombre de données disponibles peuvent également être pris en compte pour déterminer le quantile de référence $u_0$. Sur une base empirique, les présents inventeurs ont mis en évidence la pertinence du neuvième décile, à savoir le quantile à 90%, comme quantile de référence $u_0$ pour obtenir la précision souhaitée tout en tenant compte des paramètres de la distribution, dans le cas d'espèce une distribution gaussienne (taille, données aberrantes, etc.). La sélection du quantile de référence $u_0$ n'implique donc pas nécessairement des étapes d'analyse ou de calcul supplémentaires puisqu'il est tout à fait possible de mettre en œuvre la première sous-étape E1) avec la valeur empirique précitée. Autrement dit, le quantile de référence $u_0$ peut être prédéterminé/présélectionné. Pour être tout à fait complet, la première sous-étape E1) pourrait être mise en œuvre avant l'étape E), dès lors que les données de la période de référence $W_0$ sont connues.

Toujours selon cette mise en œuvre particulière, lors d'une deuxième sous-étape E2), on associe à chaque distance $d_i$(m) une variable scalaire $p_{i=1,...,N+1}$ ($0<p_{i=1,...,N+1}<1$) dépendant du quantile de référence $u_0$. Autrement dit, lors de cette deuxième sous-étape E2), le quantile $u_0$ présélectionné ou sélectionné lors de la première sous-étape E1) est utilisé pour réaliser des affectations scalaires. En effet, on affecte à chacun des log($d_i$(m)) calculés à la fin de l'étape D) une variable scalaire $p_{i=1,...,N+1}$ selon le quantile de référence $u_0$ (pré)sélectionné. Par cette étape, on s'assure que toutes les données associées aux paramètres physiologiques observés puissent être classées les unes par rapport aux autres, peu importe le type de données auxquelles elles étaient liées à l'origine, regroupées par type et fusionnées (lors de l'étape F notamment). À la fin de l'étape E), on est donc en possession d'un classifieur dans lequel les données obtenues à partir des différents signaux physiologiques mesurés peuvent être comparées, classées et fusionnées.

Selon un mode de réalisation préféré, les variables scalaires $p_{i=1,...,N+1}$ ($0<p_{i=1,...,N+1}<1$) sont calculées à partir de la transformation suivante :

$$p_{i=1,...,N+1}(m) = \frac{1}{1 + e^{u_0} - \log(d_{i=1,...,N+1}(m))} \qquad (3)$$

où $p_{i=1,...,N+1}$ sont les variables scalaires, $u_0$ est le quantile de référence, $d_{i=1...N+1}$(m) sont les distances riemanniennes et les distances statistiques.

En étant ainsi calculées, les variables scalaires $p_{i=1,...,N+1}$ se présentent donc sous la forme de probabilités. L'utilisation de variables scalaires $p_{i=1,...,N+1}$ sous une telle forme permet d'en faciliter le traitement mathématique et simplifie donc la mise en œuvre du procédé de détection selon cette mise en œuvre particulière.

Lors d'une sixième étape F), on fusionne les données obtenues à l'issu de l'étape E) en effectuant une somme pondérée des variables $p_{i=1,...,N+1}$ pour obtenir des distances résultantes $d_{fusion}(m)$. Autrement dit, pour chacun des M segments temporels m, on obtient une distance $d_{fusion}(m)$ résultant de la fusion des données obtenues à la fin de l'étape E) affectées d'un poids, c'est-à-dire d'un coefficient de pondération.

Précisons qu'un avantage de la segmentation de la fenêtre d'observation en M segments temporels m est de préserver une bonne corrélation des données issues des différents types de signaux physiologiques.

En outre, si la fusion des données en tant que telle permet de réduire les données et confère donc une plus grande efficacité algorithmique, elle est aussi très performante en ce qu'elle permet de déduire très précisément un changement de l'état physiologique du patient. En effet, chaque distance fusionnée $d_{fusion}(m)$ prend en compte les différents types de signaux physiologiques mesurés et permet d'intégrer l'information contenue dans ces différents signaux. Au lieu de superposer les mesures, éventuellement de différents capteurs, d'un même type de signal physiologique comme cela a été fait dans l'art antérieur, il s'agit de superposer et coupler les mesures de différents types de signaux physiologiques. Dans le cadre de l'invention, le fait de « fusionner » les mesures ne renvoie pas au simple fait d'apparier les mesures, c'est-à-dire de les considérer à deux, à trois, etc. Le fait de « fusionner ou combiner » doit s'entendre comme le fait de générer un résultat, ici $d_{fusion}(m)$, par la transformation mathématique des données issues des mesures, les données étant les distances $d_i(m)$ préalablement calculées. Toute détection d'un changement de l'état physiologique du patient est donc le résultat d'une combinaison/fusion des données issues des différents paramètres physiologiques. Un changement de l'état physiologique du patient établi sur la base du procédé selon l'invention signifie que plusieurs modifications homéostatiques, au moins 2, ont donc eu lieu. Il est donc plus représentatif de l'état général du patient.

Le fait d'effectuer des sommes pondérées des variables scalaires $p_{i=1,...,N+1}$ $(0<p_{i=1,...,N+1}<1)$, c'est-à-dire de sommer les variables scalaires $p_{i=1,...,N+1}$, préalablement affectées d'un coefficient de pondération, permet d'améliorer substantiellement la précision des distances fusionnées $d_{fusion}(m)$. En effet, il est possible d'attribuer un poids moindre aux données issues des signaux les plus contaminés, par exemple par le bruit, les artéfacts, etc., et au contraire d'attribuer un poids plus important aux signaux de meilleur qualité. Dans ce cas, le coefficient de pondération est défini sur la base d'un critère a postériori.

Il est aussi possible de définir un coefficient de pondération sur la base d'un critère a priori. Par exemple, il est possible d'attribuer un coefficient de pondération selon l'importance du signal $S_i$ auquel il est associé pour déterminer le changement d'état physiologique du patient, c'est-à-dire selon l'importance du paramètre physiologique observé dans le changement d'état physiologique du patient. Un critère a priori présente l'avantage d'objectiver le calcul de $d_{fusion}(m)$ en comparaison d'un critère a postériori. Toutefois, rien n'empêche d'utiliser un coefficient de pondération résultant d'un critère a priori et d'un critère a postériori. Le type de coefficient de pondération sélectionné n'est pas limitatif dans le cadre de la présente invention. D'ailleurs, il est possible d'attribuer un coefficient de pondération identique à toutes les distances les variables scalaires $p_{i=1,...,N+1}$ calculées, ce qui revient à donner une importance identique à tous les signaux mesurés.

Tous les avantages précédemment vus concourent à améliorer la détection d'un changement d'état physiologique du patient survient. Le procédé de détection selon l'invention n'en est que plus fiable et plus performant en comparaison des procédés jusqu'ici connus de l'art antérieur.

Selon une mise en œuvre préférentielle de la présente invention, $d_{fusion}(m)$ est calculée de la manière suivante :

$$d_{fusion}(m) = \sum_i \lambda_i p_{i=1,...,N+1}(m) \qquad (4)$$

où $\lambda_i$ est le poids affecté à chaque variable $p_{i=1,...,N+1}$. En ce qui concerne les variables scalaires $p_{i=1,...,N+1}$, on pourra par exemple les calculer à partir de la formule (3) précédemment décrite. Les avantages précités ne sont pas rappelés. Toutefois, on précise qu'il est préférable que la somme des coefficients de pondération $\lambda_i$ soit telle que $\lambda_1 + \lambda_2 + .... + \lambda_N = 1$. Ceci a pour but de simplifier le traitement mathématique des données dans cette mise en œuvre particulière du procédé de détection selon l'invention.

Une fois les distances $d_{fusion}(m)$ calculées, on détermine un écart e(m) à l'état physiologique de référence en fonction des distances $d_{fusion}(m)$ lors d'un septième étape G). Comme cela a été vu précédemment, cela est rendu possible dans la mesure où l'état physiologique de référence, auquel est associée la matrice de référence $X_{ref}$ de composantes $X_{ref,i}$, est déjà préalablement connu. En ce qui concerne la détermination de l'écart e(m) à l'état physiologique de référence, on peut en outre préciser que tout écart n'est pas nécessairement considéré comme reflétant un changement d'état. En effet, on peut définir un seuil $\Theta$ à partir duquel un écart e(m) par rapport à l'état physiologique de référence est considéré comme suffisamment significatif pour considérer qu'il y a effectivement un changement de l'état physiologique du patient. L'écart e(m) qui est calculé est celui entre les distances fusionnées $d_{fusion}(m)$ aux instants t : t+W et les distances fusionnées.

Selon une mise en œuvre particulière du procédé selon l'invention, préalablement à l'étape G, on effectue un filtrage des données fusionnées. Cette étape de filtrage consiste en un lissage obtenu par une moyenne mobile sur les L derniers segments temporels m de la fenêtre d'observation (1<L<M) appliquée aux distances résultantes $d_{fusion}(m)$. Le lissage vise à réduire encore plus le nombre de fausses alarmes du dispositif d'assistance respiratoire en réduisant les irrégularités généralement occasionnées par l'atténuation du signal. De préférence, cette étape de lissage est mise en œuvre en appliquant la relation suivante à $d_{fusion}(m)$ :

$$d^L_{fusion}(m) = \frac{1}{L} \sum_{i=0}^{L-1} d_{fusion}(m-i) \qquad (5)$$

La valeur de la distance de fusion ainsi calculée permet d'augmenter significativement les performances du procédé. En effet, le lissage des distances fusionnées $d_{fusion}(m)$ fournit une meilleure classification globale en réduisant leur variabilité, et ce, pour l'ensemble des M segments temporels.

Avantageusement, les L derniers segments sont choisis de telle sorte que la moyenne mobile soit calculée en ne tenant compte des signaux mesurés qu'avec un retard compris entre 10 secondes et 1 minute, de préférence compris entre 30 secondes et 1 minute par rapport au début de la fenêtre d'observation. En allongeant ainsi la fenêtre temporelle sur laquelle sont lissées les fenêtres, notamment jusqu'à environ 1 minute, il est possible d'améliorer considérablement les performances du procédé de détection selon cette mise en œuvre particulière de l'invention comme ceci sera vu plus en détail dans la suite.

On rappelle que le procédé de détection selon l'invention peut être mis en œuvre en utilisant tout autre traitement de données que celui présenté décrit précédemment en relation avec les étapes B) à F). En effet, dans le procédé selon l'invention, ce qui importe est de fusionner les données issues des mesures réalisées à partir des différents types de signaux. En outre, si selon un mode de réalisation préféré, cette fusion est mise en œuvre en effectuant une somme pondérée des données obtenues à la fin des étapes B) à E) dans le but d'obtenir des distances résultantes $d_{fusion}(m)$, un autre processus de fusion basé sur d'autres données que des distances peut être envisagé. Autrement dit, la mise en œuvre spécifique précédemment décrite en référence aux étapes B) à F) n'est proposée qu'à la seule fin de faciliter la compréhension de l'invention. On pourrait envisager de fusionner les données issues des différentes mesures par des méthodes connues par l'homme versé dans l'art telles que les méthodes basées sur les probabilités de Bayes, la théorie des croyances de Dempster-Shafer, des modèles de croyance transférables, la théorie des possibilités de Dubois et Prade, etc.

Application concrète du procédé de détection d'un changement d'état physiologique d'un patient

Dans cet exemple de réalisation, on met en œuvre le procédé de détection d'un changement d'état physiologique selon l'invention pour deux sujets SU1 et SU2 à partir des mesures de signaux $S_1$ électro-encéphalographiques et des mesures d'un signal $S_2$ respiratoire de chaque sujet. Dans cet exemple de réalisation, on a procédé comme vu précédemment en mettant en œuvre les étapes successives A à G selon une mise en œuvre particulière du procédé selon l'invention. Les figures 2a et 2b illustrent les performances obtenues avec le procédé de détection selon l'invention pour un sujet SU1 (figure 2a) et un sujet SU2 (figure 2b).

Sur chacune des figures 2a, 2b, la première figure illustre les distances riemanniennes $d_{1,r}$ estimées à partir des signaux $S_1$ électro-encéphalographiques seuls tandis que la deuxième figure illustre les distances statistiques $d_2$ estimées à partir du signal $S_2$ respiratoire seul. Autrement dit, on reporte sur ces deux premières figures les performances associées à un détecteur indépendant basé sur l'analyse d'un unique type de signal parmi les types de signaux précités. Sur chaque courbe des figures, on distingue un premier segment m1 et un deuxième segment m2 dans lesquels ont été calculées les distances riemanniennes et statistiques (correspondant aux deux états physiologiques d'un patient). Sur chacune des figures 2a, 2b, la troisième figure illustre quant à elle les distances $d_{fusion}$ obtenues après avoir procédé à la fusion de log $d_{1,r}$ et de log $d_{2,s}$ selon la formule (4) ci-dessus. On a opté pour le quantile à 90% - neuvième décile - pour définir la précision. Ici, les coefficients de pondération sont identiques $\lambda_1 = \lambda_2 = 0.5$.

Sur chaque figure (sous-figure) est indiquée une valeur correspondant à l'aire sous la courbe AUC *(Area Under the Curve* en anglais) qui permet de suivre les performances de la détection. En ce qui concerne le sujet SU1, les valeurs d'AUC associées aux distances calculées à partir des signaux $S_1$ électro-encéphalographiques seuls, à partir des signaux $S_2$ respiratoires seuls et des distances $d_{fusion}$ résultant de la fusion de ces deux types de données sont respectivement égales à 0,85, 0,74 et 0,91. L'augmentation nette de l'AUC après fusion des données correspond à une amélioration nette des performances. En ce qui concerne le sujet SU2, les valeurs d'AUC associées aux distances calculées à partir des signaux $S_1$ électro-encéphalographiques seuls, à partir des signaux $S_2$ respiratoires seuls et des distances $d_{fusion}$ résultant de la fusion de ces deux types de donnée sont respectivement égales à 0,91, 0,72 et 0,91. Dans ce cas, même si l'augmentation de l'AUC n'est pas aussi significative que ce qui a été vu pour le sujet SU1, on observe une

# EP 4 412 517 B1

amélioration de la qualité des données fusionnées en comparaison des données non fusionnées.

Sur chacune des figures 2a, 2b, la quatrième figure illustre les caractéristiques de fonctionnement du récepteur, notée ROC *(Receiver Operating Characteristic* en anglais) pour chacun des sujets. Sur chacune des figures, on distingue trois courbes correspondant, de la plus claire à la plus foncée, à la courbe ROC associée au signal $S_2$ respiratoire seul, à la courbe ROC associée aux signaux $S_1$ électro-encéphalographiques seuls et à la courbe ROC associée aux distances $d_{fusion}$ résultant de la fusion des données issues de ces deux types de mesures. On constate que le taux de fausses alarmes augmente moins rapidement avec un détecteur exploitant les distances fusionnées $d_{fusion}$ en comparaison de ce qui est mesuré par les détecteurs indépendants, c'est-à-dire les détecteurs exploitant les signaux $S_1$ électro-encéphalographiques seuls ou le signal $S_2$ respiratoire seul. On peut remarquer que pour le sujet SU2, le détecteur indépendant exploitant les signaux $S_1$ électro-encéphalographiques seuls présente néanmoins des performances comparables avec celle du détecteur exploitant les données fusionnées.

Comme ceci avait été mentionné dans le mode général de réalisation, il est également possible d'améliorer les performances des détecteurs en procédant à un filtrage des données. Si dans le procédé selon l'invention, cette étape préférentielle de filtrage est appliquée aux données fusionnées, elle peut aussi être appliquée à des données non fusionnées. Les inventeurs ont mis en œuvre ce lissage sur les données fusionnées et non fusionnées afin de mettre en évidence les performances de la fusion. Ceci est illustré sur la figure 3.

Une amélioration des performances de chacun des détecteurs peut déjà être observée en effectuant un lissage sur les données obtenues à partir de 15 secondes (i.e. avec 15 secondes de retard). En effet, comme on peut le constater sur les figures 3a, 3b et 3c respectivement, les valeurs d'AUC associées à chaque détecteur augmentent à mesure que ce délai augmente. Si une amélioration des performances apparaît clairement pour les détecteurs exploitant le signal $S_2$ respiratoire seul et les signaux $S_1$ électro-encéphalographiques seuls, elle est encore plus nette pour le détecteur exploitant les données fusionnées. Les résultats sont remarquables comme ceci apparaît sur la figure 3c).

Description d'un mode général de réalisation d'un dispositif de surveillance d'un patient selon l'invention

Il convient de noter en premier lieu que par dispositif de surveillance d'un patient on entend tout dispositif tel que décrit dans la suite dès lors qu'il permet de mesurer au moins deux paramètres physiologiques d'un patient, c'est-à-dire au moins deux fonctions et/ou réactions du patient.

L'invention concerne en outre un dispositif de surveillance d'un patient permettant la mise en œuvre d'un procédé de détection tel que précédemment décrit. Le dispositif de surveillance est avantageusement équipé d'un processeur permettant classiquement d'exécuter des tâches, dont du traitement des données, et de communiquer avec d'autres équipements auquel il est relié et/ou connecté.

Afin de mettre en œuvre le procédé selon l'invention, le dispositif de surveillance du patient comprend des moyens de mesure de signaux $S_1$ électro-encéphalographiques. Ces moyens de mesure comprennent classiquement un casque muni de n électrodes positionnées de manière appropriée au niveau de la tête du patient. Ces moyens peuvent en outre comprendre tout autre équipement permettant de réaliser des mesures électro-encéphalographiques. Le casque est relié au dispositif de surveillance et lui communique toutes les données mesurées par les électrodes. Le dispositif de surveillance est apte à recevoir, traiter les informations reçues du casque et, lorsque c'est nécessaire, d'adapter l'assistance ventilatoire qui est fournie au patient.

Le dispositif de surveillance comprend en outre des moyens de mesure de l'activité cardiaque et/ou de l'activité respiratoire et/ou de l'activité musculaire te/ou du mouvement. À cet égard, le dispositif de surveillance est relié et/ou connecté aux capteurs et autres outils de diagnostics usuellement utilisés pour effectuer les mesures des paramètres physiologiques concernés. Ces capteurs ou autres outils auxquels il est fait référence peuvent consister en des électrodes cervicales ou encore des accéléromètres selon le type de paramètre physiologique observé.

Comme précédemment indiqué, le dispositif de surveillance est apte à effectuer des tâches de traitement de données. Il comprend des moyens de traitement de signal en temps réel, notamment des moyens de détermination des composantes $X_{i,ref}$ de la matrice de référence, des moyens de calcul des écarts e(m) à la situation de référence et des moyens de fusion des données. En tout état de cause, il est apte à mettre en œuvre le procédé faisant l'objet de l'invention.

L'invention concerne en outre un dispositif d'assistance médicale équipé d'un dispositif de surveillance tel que précédemment décrit.

## Revendications

1. Procédé de détection d'un changement d'état physiologique d'un patient par rapport à un état physiologique de référence associé à une matrice de référence $X_{ref}$ de composantes $X_{ref,i}$ (i = [1... N+1], N entier) et à une période de référence $W_0$, le procédé étant mis en oeuvre par un processeur et mettant en oeuvre en boucle les étapes suivantes :

A) effectuer dans M segments temporels m d'une fenêtre d'observation des mesures de signaux $S_1$ électro-encéphalographiques du patient selon n voies et à p instants pour générer M matrices de mesures $X_{1,m}$ (m $\varepsilon$ [1... M], M entier) comportant chacune n x p échantillons et simultanément, effectuer des mesures d'au moins N autres types de signaux physiologiques $S_{N+1}$ (N ≥ 1) du patient, différent(s) des signaux $S_1$ électro-encéphalographiques, pour générer N autres matrices de mesures $X_{i = 2 ...N+1,m}$,

C) pour chaque segment temporel m de la période de référence $W_0$, déterminer des distances $d_i(m)$ entre chaque signal $X_{i,m}$ mesuré et la composante $X_{ref,i}$ de la période de référence,

D) transformer les distances $d_i(m)$ calculées à l'étape C) via une fonction log $d_i(m)$,

E) choisir la précision des valeurs obtenues à l'issue de l'étape D) en sélectionnant un quantile de référence $u_0$ sur l'ensemble des valeurs de la période de référence $W_0$ et associer à chaque distance $d_i(m)$ une variable scalaire $p_{i=1,...,N+1}$ ($0 < p_{i=1,...,N+1} < 1$) dépendant du quantile de référence $u_0$,

F) fusionner les données obtenues à l'issu de l'étape E) en effectuant une somme pondérée des variables $p_{i=1,...,N+1}$ pour obtenir des distances résultantes $d_{fusion}(m)$,

G) déterminer un écart e(m) à l'état physiologique de référence en fonction des distances $d_{fusion}(m)$.

**2.** Procédé selon la revendication 1, dans lequel lors d'une étape B) on centre et on filtre les signaux $S_1$ électro-encéphalographiques dans Q bandes de fréquence prédéterminées pour obtenir MxQ matrices de mesures filtrées $X_{1,m,q}$ (q $\varepsilon$ [1... Q]) et on détermine MxQ matrices normées de covariance spatiale par la formule :

$$C_{m,q} = \frac{X_{1,m,q}X_{1,m,q}^T}{trace\ (X_{1,m,q}X_{1,m,q}^T)}$$ et dans lequel lors de l'étape C), on détermine des distances riemanniennes $d_{i,r}$

(m) entre chaque matrice normée de covariance spatiale $C_{m,q}$ et les composantes $X_{ref,i}$ de la matrice de référence associées aux mesures électro-encéphalographiques.

**3.** Procédé selon l'une quelconque des revendications 1 à 2, dans lequel lors de l'étape C), on détermine des distances statistiques $d_{i,s}(m)$ entre un(des) signal(aux) $X_{i,m}$ mesuré(s), autres que les signaux électro-encéphalographiques, et la(les) composante(s) $X_{ref,i}$ de la période de référence qui lui est(sont) associée(s).

**4.** Procédé selon l'une quelconque des revendications précédentes, dans lequel $d_{fusion}(m)$ est calculé selon la relation suivante :

$$d_{fusion}(m) = \sum_i \lambda_i p_{i=1,...,N+1}(m),\ \text{avec}$$

$$p_{i=1,...,N+1}(m) = \frac{1}{1+e^{u_0 - \log(d_{i=1,...,N+1}(m))}}$$

où $\lambda_i$ est le poids affecté à chaque variable $p_{i=1,...,N+1}$, les $\lambda_i$ vérifiant $\sum_i \lambda_i = 1$, $u_0$ est le quantile de référence, $d_{1,q}(m)$ sont les distances riemanniennes et $d_{i>1}(m)$ sont les distances statistiques.

**5.** Procédé selon la revendication 4, dans lequel les poids $\lambda_i$ sont sélectionnés selon la qualité du signal $S_i$ auquel ils sont associés ou selon l'importance du signal $S_i$ auquel ils sont associés pour déterminer un changement d'état physiologique chez le patient.

**6.** Procédé selon l'une quelconque des revendications précédentes, dans lequel une étape de filtrage des distances résultantes $d_{fusion}(m)$ est mise en oeuvre préalablement à l'étape G).

**7.** Procédé selon la revendication 6, dans lequel le filtrage consiste en un lissage obtenu par une moyenne mobile sur les L derniers segments de la fenêtre d'observation (1<L<M) appliquée aux distances résultantes $d_{fusion}(m)$, ladite distance $d_{fusion}(m)$ étant alors calculée selon la relation suivante :

$$d_{fusion}^L(m) = \frac{1}{L}\sum_{i=0}^{L-1} d_{fusion}(m-i)$$

**8.** Procédé selon la revendication 7, dans lequel les L derniers segments sont choisis de telle sorte que la moyenne mobile soit calculée en ne tenant compte des signaux mesurés qu'avec un retard compris entre 10 secondes et 1 minute, de préférence compris entre 30 secondes et 1 minute par rapport au début de la fenêtre d'observation.

9. Procédé selon l'une quelconque des revendications précédentes, dans lequel lesdits autres signaux physiologiques $S_i$ sont l'activité cardiaque et/ou l'activité respiratoire et/ou l'activité musculaire et/ou un signal lié au mouvement.

10. Procédé selon l'une quelconque des revendications précédentes, comprenant en outre une étape au cours de laquelle on compare chacun des écarts e(m) à un seuil $\Theta$ déterminé.

11. Dispositif de surveillance d'un patient pour la mise en œuvre d'un procédé selon l'une quelconque des revendications précédentes, comportant :

- des moyens de mesure de signaux électro-encéphalographique pour mesurer des signaux $S_1$ électro-encéphalographiques du patient,
- des moyens de mesure de l'activité cardiaque et/ou de l'activité respiratoire et/ou de l'activité musculaire et/ou d'un mouvement,
- des moyens de traitement de signal en temps réel, lesdits moyens comprenant au moins des moyens de détermination des composantes $X_{i,ref}$ de la période de référence, des moyens de calcul des écarts e(m) à la situation de référence et des moyens de fusion des données,
- un processeur permettant de mettre en œuvre le procédé de détection d'un changement d'état physiologique d'un patient par rapport à un état physiologique de référence selon l'une quelconque des revendications 1 à 10.

12. Dispositif d'assistance médicale comportant un dispositif de surveillance selon la revendication 11.

**Patentansprüche**

1. Verfahren zur Erkennung einer Veränderung des physiologischen Zustands eines Patienten in Bezug auf einen physiologischen Referenzzustand, der einer Referenzmatrix $X_{ref}$ aus Komponenten $X_{ref,i}$ (i = [1... N+1], N ganzzahlig) und einer Referenzperiode $W_0$ zugeordnet ist, wobei das Verfahren durch einen Prozessor implementiert wird und die folgenden Schritte in einer Schleife implementiert:

A) Ausführen in M Zeitsegmenten m eines Beobachtungsfensters von Messungen elektroenzephalographischer Signale $S_1$ des Patienten gemäß $n$ Kanälen und zu $p$ Zeitpunkten, um M Messmatrizen $X_{1,m}$ (m $\varepsilon$ [1... M], M ganzzahlig) zu erzeugen, die jeweils n x p Abtastwerte umfassen, und gleichzeitig Ausführen von Messungen von mindestens N anderen Arten von physiologischen Signalen $S_{N+1}$ (N $\geq$ 1) des Patienten, die sich von den elektroenzephalographischen Signalen $S_1$ unterscheiden, um N weitere Messmatrizen $X_{1=2..N+1,m}$ zu erzeugen,
C) für jedes Zeitsegment m der Referenzperiode $W_0$, Bestimmen der Abstände $d_i(m)$ zwischen jedem gemessenen Signal $X_{i,m}$ und der Komponente $X_{ref,i}$ der Referenzperiode,
D) Umwandeln der in Schritt C) berechneten Abstände $d_i(m)$ über eine Funktion log $d_i(m)$,
E) Auswählen der Genauigkeit der aus dem Schritt D) erhaltenen Werte durch Selektieren eines Referenzquantils $u_0$ aus der Gesamtheit der Werte der Referenzperiode $W_0$ und Zuordnen zu jedem Abstand $d_i(m)$ einer skalaren Variablen $p_{i=1,...,N+1}$ ($0<p_{i=1,..,N+1}<1$) abhängig vom Referenzquantil $u_0$,
F) Zusammenführen der aus dem Schritt E) erhaltenen Daten durch Ausführen einer gewichteten Summe der Variablen $P_{i=1,...,N+1}$, um die resultierenden Abstände $d_{fusion}(m)$ zu erhalten,
G) Bestimmen einer Abweichung e(m) vom physiologischen Referenzzustand in Abhängigkeit von den Abständen $d_{fusion}(m)$.

2. Verfahren nach Anspruch 1, wobei während eines Schritts B) die elektroenzephalographischen Signale Si in Q vorbestimmten Frequenzbändern zentriert und gefiltert werden, um MxQ gefilterte Messmatrizen $X_{1,m,q}$ (q $\varepsilon$ [1...Q]) zu erhalten und MxQ normierte räumliche Kovarianzmatrizen durch die Formel bestimmt werden:

$$C_{m,q} = \frac{X_{1,m,q}X_{1,m,q}{}^T}{Spur\left(X_{1,m,q}X_{1,m,q}{}^T\right)}$$ und wobei während des Schritts C) Riemannsche Abstände $d_{1,r}(m)$ zwischen jeder normierten räumlichen Kovarianzmatrix $C_{m,q}$ und den Komponenten $X_{ref,i}$ der Referenzmatrix, die den elektroenzephalographischen Messungen zugeordnet sind, bestimmt werden.

3. Verfahren nach einem der Ansprüche 1 bis 2, wobei während des Schritts C) statistische Abstände $d_{i,s}(m)$ zwischen einem oder mehreren gemessenen Signal(en) $X_{i,m}$, die keine elektroenzephalographischen Signale sind, und der oder den Komponent(en) $X_{ref,i}$ der Referenzperiode, die ihnen zugeordnet ist/sind, bestimmt werden.

4. Verfahren nach einem der vorstehenden Ansprüche, wobei d$_{fusion}$(m) gemäß der folgenden Beziehung berechnet wird:

$$d_{fusion}(m) = \sum_i \lambda_i p_{i=1,...,N+1}(m), \text{mit}$$

$$p_{i=1,...,N+1}(m) = \frac{1}{1 + e^{u_0} - \log(d_{i=1,...,N+1}(m))}$$

wobei $\lambda_i$ die Gewichtung ist, die jeder Variablen $p_{i=1,...,N+1}$ zugewiesen wird, wobei die $\lambda_i \sum_i \lambda_i = 1$ verifiziert, $u_0$ das Referenzquantil ist, d$_{1,q}$(m) die Riemannschen Abstände sind und d$_{i>1}$(m) die statistischen Abstände sind.

5. Verfahren nach Anspruch 4, wobei die Gewichtungen $\lambda_i$ entsprechend der Qualität des Signals S$_i$, dem sie zugeordnet sind, oder entsprechend der Bedeutung des Signals S$_i$, dem sie zugeordnet sind, ausgewählt werden, um eine Veränderung des physiologischen Zustands des Patienten zu bestimmen.

6. Verfahren nach einem der vorstehenden Ansprüche, wobei ein Filterungsschritt der resultierenden Abstände d$_{fusion}$(m) vor dem Schritt G) implementiert wird.

7. Verfahren nach Anspruch 6, wobei die Filterung aus einer Glättung besteht, die durch ein gleitendes Mittel über die letzten L Segmente des Beobachtungsfensters (1<L<M) erhalten wird, der auf die resultierenden Abstände d$_{fusion}$(m) angewendet wird, wobei der Abstand d$_{fusion}$(m) dann gemäß der folgenden Beziehung berechnet wird:

$$d_{fusion}^{L}(m) = \frac{1}{L} \sum_{i=0}^{L-1} d_{fusion}(m-i)$$

8. Verfahren nach Anspruch 7, wobei die L letzten Segmente so ausgewählt werden, dass das gleitende Mittel berechnet wird, indem nur die gemessenen Signale mit einer Verzögerung im Bereich von 10 Sekunden bis 1 Minute, vorzugsweise im Bereich von 30 Sekunden bis 1 Minute, in Bezug auf den Beginn des Beobachtungsfensters, berücksichtigt werden.

9. Verfahren nach einem der vorstehenden Ansprüche, wobei die anderen physiologischen Signale S$_i$ die Herzaktivität und/oder die Atmungsaktivität und/oder die Muskelaktivität und/oder ein bewegungsbezogenes Signal sind.

10. Verfahren nach einem der vorstehenden Ansprüche, das weiter einen Schritt umfasst, bei dem jede der Abweichungen e(m) mit einem bestimmten Schwellenwert $\Theta$ verglichen wird.

11. Vorrichtung zur Überwachung eines Patienten zur Implementierung eines Verfahrens nach einem der vorstehenden Ansprüche, umfassend:

- Mittel zum Messen elektroenzephalographischer Signale, um elektroenzephalographische Signale S$_1$ des Patienten zu messen,
- Mittel zum Messen der Herzaktivität und/oder der Atmungsaktivität und/oder der Muskelaktivität und/oder einer Bewegung,
- Mittel zur Echtzeit-Signalverarbeitung, wobei die Mittel mindestens Mittel zur Bestimmung der Komponenten X$_{i,ref}$ der Referenzperiode, Mittel zur Berechnung der Abweichungen e(m) von der Referenzsituation und Mittel zur Datenzusammenführung umfassen,
- einen Prozessor, der die Implementierung des Verfahrens zur Erkennung einer Veränderung des physiologischen Zustands eines Patienten in Bezug auf einen physiologischen Referenzzustand nach einem der Ansprüche 1 bis 10 ermöglicht.

12. Medizinische Hilfsvorrichtung, die eine Überwachungsvorrichtung nach Anspruch 11 umfasst.

**Claims**

1. A method for detecting a change in the physiological condition of a patient relative to a reference physiological condition associated with a reference matrix $X_{ref}$ with components $X_{ref,i}$ ($i = [1...N+1]$, N integer) and with a reference period $W_0$, the method being a processor-implemented method and implementing the following steps in a loop:

   A) in M time segments m of an observation window, performing measurements of electroencephalographic signals $S_1$ from the patient along $n$ paths and at $p$ times to generate M measurement matrices $X_{1,m}$ ($m \, \varepsilon \, [1....M]$, M integer) each comprising n*p samples, and simultaneously performing measurements of at least N other types of physiological signals $S_{N+1}$ ($N \geq 1$) from the patient, different from the electroencephalographic signals $S_1$, to generate N other measurement *matrices $X_{i = 2..N+1,m}$,*
   C) for each time segment m of the reference period $W_0$, determining distances $d_i(m)$ between each measured signal $X_{i,m}$ and the component $X_{ref,i}$ of the reference period,
   D) transforming the distances $d_i(m)$ calculated in step C) using a log $d_i(m)$ function,
   E) choosing the precision of the values obtained at the end of step D) by selecting a reference quantile $u_0$ from all the values of the reference period $W_0$ and associating with each distance $d_i(m)$ a scalar variable $p_{i=1,...,N+1}$ ($0<p_{i=1,...,N+1}<1$) depending on the reference quantile $u_0$,
   F) merging the data obtained in step E) by performing a weighted sum of the variables $p_{i=1,...,N+1}$ to obtain the resulting distances $d_{fusion}(m)$,
   G) determining a deviation e(m) from the physiological reference condition as a function of the distances $d_{fusion}(m)$.

2. The method according to claim 1, wherein in a step B) the electroencephalographic signals $S_1$ are centred and filtered in Q predetermined frequency bands to obtain MxQ filtered measurements matrices $X_{1,m,q}$ ($q \, \varepsilon \, [1...Q]$) and MxQ normalised spatial covariance matrices are determined by the formula:

$$C_{m,q} = \frac{X_{1,m,q}X_{1,m,q}{}^T}{trace \, (X_{1,m,q}X_{1,m,q}{}^T)}$$

   and in which, in step C), Riemannian distances $d_{i,r}(m)$ are determined between each normalised spatial covariance matrix $C_{m,q}$ and the components $X_{ref,i}$ of the reference matrix associated with the electroencephalographic measurements.

3. The method according to any one of claims 1 to 2, wherein, during step C), statistical distances $d_{i,s}(m)$ are determined between one or more measured signals $X_{i,m}$ other than the electroencephalographic signals, and the component or components $X_{ref,i}$ of the reference period associated with it.

4. The method according to any one of the preceding claims, wherein $d_{fusion}(m)$ is calculated according to the following relationship:

$$d_{fusion}(m) = \textstyle\sum_i \lambda_i p_{i=1,...,N+1}(m), \text{ with}$$

$$p_{i=1,...,N+1}(m) = \frac{1}{1+e^{u_0-\log(d_{i=1,...,N+1}(m))}}$$

   where $\lambda_i$ is the weight assigned to each variable $p_{i=1,...,N+1}$, the $\lambda_i$ verifying $\sum_i \lambda_i = 1$, $u_0$ is the reference quantile, $d_{1,q}(m)$ are the Riemannian distances and $d_{i>1}(m)$ are the statistical distances.

5. The method according to claim 4, wherein the weights $\lambda_i$ are selected according to the quality of the signal $S_i$ with which they are associated or according to the importance of the signal $S_i$ with which they are associated for determining a change in physiological condition in the patient.

6. The method according to any one of the preceding claims, wherein a step of filtering the resulting distances $d_{fusion}(m)$ is implemented prior to step G).

7. The method according to claim 6, wherein the filtering consists of a smoothing obtained by a moving average over the last L segments of the observation window (1<L<M) applied to the resulting distances $d_{fusion}(m)$, said distance $d_{fusion}(m)$ then being calculated according to the following relationship:

$$d_{fusion}^{L}(m) = \frac{1}{L}\sum_{i=0}^{L-1} d_{fusion}(m-i)$$

8.  The method according to claim 7, wherein the last L segments are chosen so that the moving average is calculated taking account of the measured signals only with a delay of between 10 seconds and 1 minute, preferably between 30 seconds and 1 minute, relative to the start of the observation window.

9.  The method according to any one of the preceding claims, wherein said other physiological signals $S_i$ are cardiac activity and/or respiratory activity and/or muscular activity and/or a movement-related signal.

10. The method according to any one of the preceding claims, further comprising a step during which each of the deviations e(m) is compared with a determined threshold $\Theta$.

11. A device for monitoring a patient for the implementation of a method according to any one of the preceding claims, comprising:

    - electroencephalographic signal measuring means for measuring the patient's electroencephalographic signals $S_1$,
    - means for measuring cardiac activity and/or respiratory activity and/or muscular activity and/or movement,
    - real-time signal processing means, said means comprising at least means for determining the components $X_{i,ref}$ of the reference period, means for calculating the deviations e(m) from the reference situation and means for merging the data,

      - a processor able to implement the method for detecting a change in the physiological condition of a patient relative to a reference physiological condition according to any one of claims 1 to 10.

12. A medical assistance device comprising a monitoring device according to claim 11.

Fig. 1

Fig. 2a

Fig. 2b

Fig. 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5820560 A
- US 2010252038 A1
- WO 2013164462 A1
- WO 2013140229 A1
- US 20040254493 A
- FR 2903314 A
- US 20040254493 A1

**Littérature non-brevet citée dans la description**

- **E. DIDAY**. Une nouvelle méthode en classification automatique et reconnaissance des formes la méthode des nuées dynamiques. *Revue de Statistique Appliquée*, 1971 (2), 19-33
- Statistical computing on manifolds for computational anatomy. **PENNEC**. Thèse de doctorat. Université Nice Sophia Antipolis, 2006
- **BASSEVILLE, M.** Distance measures for signal processing and pattern recognition. *Signal processing*, 1989, vol. 18 (4), 349-369